(19) Europäisches Patentamt
European Patent Office
Office européen des brevets

(11) **EP 4 401 083 A2**

(12) **EUROPEAN PATENT APPLICATION**

(43) Date of publication:
**17.07.2024 Bulletin 2024/29**

(21) Application number: **24150004.0**

(22) Date of filing: **02.01.2024**

(51) International Patent Classification (IPC):
***G16C 60/00*** (2019.01)

(52) Cooperative Patent Classification (CPC):
**G16C 60/00;** G16C 10/00; G16C 20/10;
G16C 20/30; G16C 20/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.01.2023 IN 202321002360**

(71) Applicant: **Tata Consultancy Services Limited
Maharashtra (IN)**

(72) Inventors:
• **DAHALE, CHINMAY**
**411013 Pune - Maharashtra (IN)**
• **GOVERAPET SRINIVASAN, SRIRAM**
**600113 Chennai, Tamil Nadu (IN)**
• **RAI, BEENA**
**411013 Pune - Maharashtra (IN)**

(74) Representative: **Goddar, Heinz J.
Boehmert & Boehmert
Anwaltspartnerschaft mbB
Pettenkoferstrasse 22
80336 München (DE)**

(54) **SYSTEMS AND METHODS FOR IDENTIFYING MULTI COMPONENT ALLOY CATALYSTS FOR A TARGET CHEMICAL REACTION**

(57) Conventional approaches are not efficient for the screening of active multi component alloy catalysts. Present disclosure provides systems and method that incorporate elements of materials chemistry, artificial intelligence (AI)/machine learning (ML) and optimization for the screening of such active catalysts for a target chemical reaction. Given a set of elements constituting the alloy and the target reaction, surface atomic structures of several different alloy composition and configurations are constructed. Then, adsorption energy of key reaction intermediates on the surface atomic structures are computed using quantum chemical methods (QCM). Next, an AI/ML model is trained using data obtained from QCM to predict the adsorption energy of the reaction intermediates on the alloy surface. Finally, numerical optimization is used to identify alloy compositions that maximize the activity and selectivity of the catalyst for a target chemical reaction.

receiving (i) a set of elements constituting a multi component alloy and (ii) a target chemical reaction — 202

generating an initial set of alloy compositions from the set of elements constituting the multi component alloy — 204

analyzing the initial set of alloy compositions for the multi component alloy to determine an inclusion or an exclusion of segregation effect(s) in the initial set of alloy compositions — 206

iteratively optimizing the multi component alloy

predicting a set of relevant intermediate adsorption energies on surface atomic structures of the initial set of alloy compositions using trained ML models based on the inclusion or the exclusion of the segregation effects in the initial set of alloy compositions — 208a

identifying subsequent best candidate alloy composition(s) using the initial set of alloy compositions and the set of relevant predicted intermediate adsorption energies — 208b

until an optimization convergence is reached — 208

identifying at least one best candidate alloy composition amongst the subsequent best candidate alloy compositions as a multi component alloy catalyst that potentially maximizes activity and selectivity for the target chemical reaction — 210

**FIG. 2**

EP 4 401 083 A2

**Description**

CROSS-REFERENCE TO RELATED APPLICATIONS AND PRIORITY

The present application claims priority to Indian application no. 202321002360, filed on January 11, 2023.

TECHNICAL FIELD

The disclosure herein generally relates to multi component alloy catalysts, and, more particularly, to systems and methods for identifying multi component alloy catalysts for a target chemical reaction.

BACKGROUND

With an ever-increasing demand for clean energy and reduction of carbon emissions, identification of unique, active, and earth-abundant catalysts for various chemical conversions is essential. Multi component alloys are emerging as such promising candidates. However, a systematic approach to screen the exceptionally large compositional and configurational space of these alloys to identify 'active' catalysts is currently lacking. Exploration based on a purely experimental approach, or via accurate quantum chemistry calculations alone is impractical to cover this vast space. Further, conventional approaches may not be efficient and simultaneously do not incorporate elements of materials chemistry and optimization that require screening of active multi component catalysts.

SUMMARY

Embodiments of the present disclosure present technological improvements as solutions to one or more of the above-mentioned technical problems recognized by the inventors in conventional systems.

For example, in one aspect, there is provided a processor implemented method for identifying multi component alloy catalysts for a target chemical reaction. The method comprises receiving, via one or more hardware processors, (i) a set of elements constituting a multi component alloy, and (ii) a target chemical reaction; generating an initial set of alloy compositions from the set of elements constituting the multi component alloy; analyzing the initial set of alloy compositions for the multi component alloy to determine an inclusion or an exclusion of one or more segregation effects in the initial set of alloy compositions; iteratively optimizing the multi component alloy by: predicting a set of relevant intermediate adsorption energies on the one or more surface atomic structures of the initial set of alloy compositions using the one or more trained ML models based on the inclusion or the exclusion of one or more segregation effects in the initial set of alloy compositions; and identifying one or more subsequent best candidate alloy compositions using the initial set of alloy compositions and the set of relevant predicted intermediate adsorption energies, until an optimization convergence is reached; and identifying at least one best candidate alloy composition amongst the one or more subsequent best candidate alloy compositions as a multi component alloy catalyst that potentially maximizes activity and selectivity for the target chemical reaction.

In an embodiment, the one or more trained ML models are obtained by identifying, via the one or more hardware processors, one or more relevant reaction intermediates for the target chemical reaction; computing, by using a quantum chemical technique via the one or more hardware processors, an adsorption energy of the one or more relevant reaction intermediates on one or more surface atomic structures of the multi component alloy; and training, via the one or more hardware processors, one or more machine learning (ML) models using the adsorption energy of the one or more relevant reaction intermediates on the one or more surface atomic structures of the multi component alloy.

In an embodiment, the inclusion of the one or more segregation effects in the initial set of alloy compositions comprises simulating the one or more surface atomic structures of the multi component alloy by using an interatomic potential based hybrid monte-carlo molecular dynamic technique.

In an embodiment, the exclusion of one or more segregation effects in the initial set of alloy compositions comprises randomly arranging one or more atoms in the one or more surface atomic structures of the multi component alloy.

In another aspect, there is provided a processor implemented system for identifying multi component alloy catalysts for a target chemical reaction. The system comprises: a memory storing instructions; one or more communication interfaces; and one or more hardware processors coupled to the memory via the one or more communication interfaces, wherein the one or more hardware processors are configured by the instructions to: receive (i) a set of elements constituting a multi component alloy, and (ii) a target chemical reaction; generate an initial set of alloy compositions from the set of elements constituting the multi component alloy; analyze the initial set of alloy compositions for the multi component alloy to determine an inclusion or an exclusion of one or more segregation effects in the initial set of alloy compositions; iteratively optimize the multi component alloy by: predicting a set of relevant intermediate adsorption energies on the one or more surface atomic structures of the initial set of alloy compositions using one or more trained ML models based on the inclusion or the exclusion of one or more segregation effects in the initial set of alloy compositions; and identifying one or more subsequent best candidate alloy compositions using the initial set of alloy compositions and the set of relevant predicted intermediate adsorption energies, until an optimization convergence is reached; and identify at least one best candidate alloy composition amongst the one or more subsequent best candidate alloy compositions as a multi component alloy catalyst that potentially maximizes activity and selectivity for the target chemical reaction.

In an embodiment, the one or more trained ML models are obtained by identifying one or more relevant reaction intermediates for the target chemical reaction; computing, by using a quantum chemical technique, an adsorption energy of the one or more relevant reaction intermediates on one or more surface atomic structures of the multi component alloy; and training one or more machine learning (ML) models using the adsorption energy of the one or more relevant reaction intermediates on the one or more surface atomic structures of the multi component alloy.

In an embodiment, the inclusion of the one or more segregation effects in the initial set of alloy compositions comprises simulating the one or more surface atomic structures of the multi component alloy by using an interatomic potential based hybrid monte-carlo molecular dynamic technique.

In an embodiment, the exclusion of one or more segregation effects in the initial set of alloy compositions comprises randomly arranging one or more atoms in the one or more surface atomic structures of the multi component alloy.

In yet another aspect, there are provided one or more non-transitory machine-readable information storage mediums comprising one or more instructions which when executed by one or more hardware processors cause identifying multi component alloy catalysts for a target chemical reaction by: receiving (i) a set of elements constituting a multi component alloy, and (ii) a target chemical reaction; generating an initial set of alloy compositions from the set of elements constituting the multi component alloy; analyzing the initial set of alloy compositions for the multi component alloy to determine an inclusion or an exclusion of one or more segregation effects in the initial set of alloy compositions; iteratively optimizing the multi component alloy by: predicting a set of relevant intermediate adsorption energies on the one or more surface atomic structures of the initial set of alloy compositions using the one or more trained ML models based on the inclusion or the exclusion of one or more segregation effects in the initial set of alloy compositions; and identifying one or more subsequent best candidate alloy compositions using the initial set of alloy compositions and the set of relevant predicted intermediate adsorption energies, until an optimization convergence is reached; and identifying at least one best candidate alloy composition amongst the one or more subsequent best candidate alloy compositions as a multi component alloy catalyst that potentially maximizes activity and selectivity for the target chemical reaction.

In an embodiment, the one or more trained ML models are obtained by: identifying one or more relevant reaction intermediates for the target chemical reaction; computing, by using a quantum chemical technique, an adsorption energy of the one or more relevant reaction intermediates on one or more surface atomic structures of the multi component alloy; training the one or more machine learning (ML) models using the adsorption energy of the one or more relevant reaction intermediates on the one or more surface atomic structures of the multi component alloy.

In an embodiment, the inclusion of the one or more segregation effects in the initial set of alloy compositions comprises simulating the one or more surface atomic structures of the multi component alloy by using an interatomic potential based hybrid monte-carlo molecular dynamic technique.

In an embodiment, the exclusion of one or more segregation effects in the initial set of alloy compositions comprises randomly arranging one or more atoms in the one or more surface atomic structures of the multi component alloy.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 depicts an exemplary system for identifying multi component alloy catalysts for a target chemical reaction, in accordance with an embodiment of the present disclosure.

FIG. 2 depicts an exemplary flow chart illustrating a method for identifying multi component alloy catalysts for a target chemical reaction, using the system of FIG. 1, in accordance with an embodiment of the present disclosure.

FIG. 3 depicts a carbon monoxide (CO) and Hydrogen (H) Adsorption energy distribution on (111) random and segregated surface atomic structures of an equimolar AgAuCuPdPt alloy (also referred as random and segregated surface atomic structures of an equimolar AgAuCuPdPt alloy of orientation (111)), in accordance with an embodiment of the present disclosure.

FIGS. 4A-4B depict an evolution of one or more objective function values (also referred as optimization convergence) over the 100 Bayesian Optimization (BO) iterations for the random and segregated systems respectively, in accordance with an embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

As mentioned earlier, conventional approaches may not be efficient and simultaneously do not incorporate elements of materials chemistry and optimization that are required for screening of active multi component catalysts. Present disclosure provides systems and method that incorporate elements of materials chemistry, artificial intelligence (AI)/machine learning (ML) and optimization, for the screening of multi component alloys that are efficient catalysts for a target chemical conversion. Given a set of elements constituting the alloy and the target reaction, the systems and methods first construct surface models (also referred as surface atomic structures) of several different alloy compositions and configurations. Then, the adsorption energy of key reaction intermediates on these surfaces (also referred as surface atomic structures and interchangeably used herein) are computed using one or more quantum chemical methods (e.g., density functional theory (DFT) calculations). Next, an AI/MI, model is trained using the data obtained from DFT calculations to predict the adsorption energy of the reaction intermediates on the alloy surface. Finally, numerical optimization is used to identify alloy compositions that maximize the activity of the catalyst for the target reaction. A mathematical function of these adsorption energies is used as the objective function in numerical optimization. Since multicomponent alloys tend to undergo preferential elemental segregation, classical interatomic potential based monte-carlo/molecular mechanics (MC/MM) calculations are carried out to identify such effects in alloy compositions identified during each iteration of the optimization procedure. The AI/MI, model is then used to predict the adsorption energies on the segregated surfaces and the objective function is evaluated using these values.

In the above outlined procedure, the MC/MM step can be avoided if the goal is to screen purely random multi component alloy catalysts. In that case, the AI/ML model is directly used on the surface of alloy composition predicted in each iteration of the optimization procedure, bypassing the MC/MM step.

The present disclosure and its system and method address the following technical problems:

1. Overcomes the lack of a systematic computational framework for the screening of active catalysts for any target reaction from the vast compositional and configurational space of multi-component alloys.
2. Provides a 'chemical reaction' and 'principal alloy component' agnostic methodology for screening of these alloy catalysts.
3. Integrates aspects of materials chemistry, data driven modeling and optimization into a single unified framework for the discovery of multi component alloy catalysts.

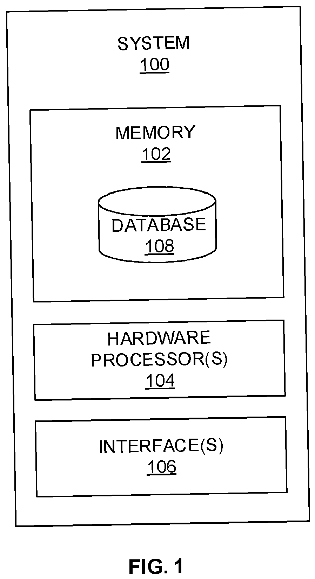

Referring now to the drawings, and more particularly to FIGS. 1 through 4B, where similar reference characters denote corresponding features consistently throughout the figures, there are shown preferred embodiments and these embodiments are described in the context of the following exemplary system and/or method.

FIG. 1 depicts an exemplary system 100 for identifying multi component alloy catalysts for a target chemical reaction, in accordance with an embodiment of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises information elements constituting a multi component alloy, and a target chemical reaction. The database 108 further comprises one or more relevant reaction intermediates for the target chemical reaction, adsorption energy of the one or more relevant reaction intermediates on one or more surface atomic structures of the multi component alloy, initial set of alloy compositions generated from the set of elements constituting the multi component alloy, analyzes of the initial set of alloy compositions for the multi component alloy, relevant intermediate adsorption energies, subsequent best candidate alloy compositions, one or more multi component alloy catalysts, and the like. The memory 102 further comprises various models such as artificial intelligence, machine learning model(s), and the like that are trained and invoked for execution and when executed enable method of predicting catalyst for target chemical reaction as described herein. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

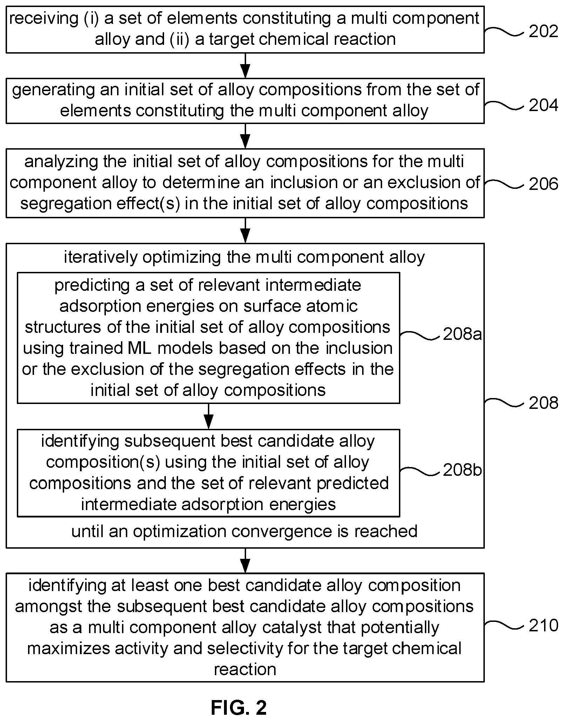

FIG. 2, with reference to FIG. 1, depicts an exemplary flow chart illustrating a method for identifying multi component alloy catalysts for a target chemical reaction, using the system 100 of FIG. 1, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, and the flow diagram as depicted in FIG. 2.

At step 202 of the method of the present disclosure, the one or more hardware processors 104 receive (i) a set of elements constituting a multi component alloy, and (ii) a target chemical reaction. For instance, the system 100 considers elements Silver (Ag), Gold (Au), Copper (Cu), Palladium (Pd), and Platinum (Pt) containing multi component alloy for carbon monoxide (CO) reduction reaction (target chemical reaction).

At step 204 of the method of the present disclosure, the one or more hardware processors 104 generate an initial set of alloy compositions from the set of elements constituting the multi component alloy, to be used as the initial guesses for the optimization of the alloy composition. The initial dataset for optimization consisted of 58 different alloys of unary, binary, ternary, quaternary as well as quinary compositions, sampled randomly. The initial set of alloy compositions is generated using one or more trained ML models.

The step of obtaining the one or more trained ML models is better understood by way of following description:

At first, the one or more hardware processors 104 identify one or more relevant reaction intermediates for the target chemical reaction (or a plurality of target chemical reactions). Identification of the one or more relevant reaction intermediates for the target chemical reaction is better understood by way of following description. Large scale deployment of CO/CO2 reduction (CORR) to value added chemicals is one of the ways to achieve a carbon neutral economy. The major impediment towards realizing this is the development of a catalyst that is active and selective for CORR. As with any catalytic conversion, catalysts for a reaction can be qualified by using the adsorption energy of one or more key intermediates as the 'descriptor'. In the specific case of CORR, the adsorption energy of CO molecule on the catalyst surface is a widely accepted descriptor for ranking catalysts. In addition to CORR, the catalyst must be poorly active for parasitic reactions such as hydrogen evolution reaction (HER). For HER, the adsorption energy of hydrogen atom on the surface of the catalyst is usually used as the descriptor to qualify the catalyst. Hence, the task herein is to optimize the composition of the Ag, Au, Cu, Pd, Pt containing multi component alloy to maximize its activity and selectivity for CORR and minimize its activity towards HER.

Further, the one or more hardware processors 104 compute, by using a quantum chemical technique, an adsorption energy of the one or more relevant reaction intermediates on one or more surface atomic structures of the multi component alloy. Computation of the adsorption energy of the one or more relevant reaction intermediates on one or more surface atomic structures of the multi component alloy is better understood by way of following description.

The first step is to generate data for the adsorption energy of CO and H on the surface atomic structures of the multi component alloy. This data is usually generated via high throughput density function theory (DFT) calculations. In the present disclosure, the system 100 and the method took this DFT data from a recently published article available in the open literature (e.g., refer "Pedersen et al, ACS Catal. 2020, 10, 3, 2169-2176"). This DFT data consisted of the adsorption energy of CO molecule on various 'ontop' sites and H atom on various 'HCP' and 'FCC' hollow sites on the 111 surface atomic structures of the multi component alloy.

Furthermore, the one or more hardware processors 104 train one or more machine learning (ML) models (also referred as ML algorithms and interchangeably used herein) using the adsorption energy of the one or more relevant reaction intermediates on the one or more surface atomic structures of the multi component alloy. Training of the one or more machine learning (ML) models is better understood by way of following description.

More specifically, the system 100 and the method train artificial intelligence (AI)/ML models for predicting the adsorption energies of these adsorbates. While several different models of varying sophistication can be trained, the system 100 trained a ML model that used a one-hot encoded representation of the chemical environment around the binding site of both CO and H adsorbates as the features, as outlined in Pedersen et al, ACS Catal. 2020, 10, 3, 2169-2176. Eight (8) different ML algorithms were used to build ML models for each of CO and H adsorption energies. Seven different data scaling techniques were tested with each ML model. The ML models were validated using a 10-fold cross-validation procedure. GridSearchCV module of sci-kit learn as used for hyperparameter optimization. Below Tables 1 and 2 show the results from the ML model fitting to CO and H adsorbate adsorption energies. More specifically, Table 1 depicts Evaluation of the ML models employed to fit CO Adsorption energies. Table 2 depicts Evaluation of the ML models employed to fit H Adsorption energies. Kernel ridge regression (KRR) model performed best on both the regression tasks, with a very small test MAE of 0.04 and 0.05 eV respectively. Now, for any new alloy composition, these trained ML models can be used to predict the adsorption energy of the two intermediates on the (111) surface atomic structures of the multi component alloy.

Table 1

| ML Algorithm (ML model) | Scaling method | Test MAE (eV) | Test $R^2$ | Optimized hyperparameters |
|---|---|---|---|---|
| KRR (linear) | Quantile (Normal) | 0.055 | 0.9823 | $\alpha$ = 0.25 |
| **KRR (RBF)** | **MinMaxScaler** | **0.040** | **0.9921** | $\alpha$ **= 0.01, $\gamma$ = 0.048** |
| KRR (Laplacian) | Quantile (Uniform) | 0.041 | 0.9916 | $\alpha$ =0.08, $\gamma$ = 0.01 |
| KRR (Polynomial) | PowerTransformer | 0.041 | 0.9920 | $\alpha$ = 0.07, $\gamma$ = 0.01, degree = 3 |
| Random Forest | PowerTransformer | 0.045 | 0.9894 | n_estimators = 55, max_depth = 8, min_ sample_leaf = 1 |
| AdaBoost | MinMaxScaler | 0.057 | 0.9836 | n_estimators = 43, loss = square |
| XgBoost | Quantile (Uniform) | 0.043 | 0.9910 | n_estimators = 92, loss = huber |
| KNN | MinMaxScaler | 0.051 | 0.9850 | n_neighbors=7, p = 1 |

Table 2

| ML Algorithm | Scaling method | Test MAE (eV) | Test $R^2$ | Optimized hyperparameters |
|---|---|---|---|---|
| KRR (linear) | RobustScaler | 0.059 | 0.8930 | $\alpha$ = 0.05 |
| KRR (RBF) | Quantile (Uniform) | 0.060 | 0.8913 | $\alpha$ = 0.01, $\gamma$ = 0.01 |
| **KRR (Laplacian)** | **StandardScaler** | **0.059** | **0.8933** | $\alpha$ **=0.03, $\gamma$ = 0.01** |
| KRR (Polynomial) | MinMaxScaler | 0.059 | 0.8930 | $\alpha$ = 0.01, $\gamma$ = 0.181, degree = 1 |
| Random Forest | MaxAbsScaler | 0.064 | 0.8798 | n_estimators = 54, max_depth = 33, min_sample_ leaf = 1 |
| AdaBoost | RobustScaler | 0.16 | 0.3581 | n_estimators = 40, loss = square |
| XgBoost | RobustScaler | 0.060 | 0.8827 | n_estimators = 197, loss = ls |
| KNN | MinMaxScaler | 0.067 | 0.8712 | n_neighbors=4, p = 2 |

Referring to steps of FIG. 2, at step 206 of the method of the present disclosure, the one or more hardware processors 104 analyze the initial set of alloy compositions for the multi component alloy to determine an inclusion or an exclusion of one or more segregation effects in the initial set of alloy compositions. More specifically, at step 206, it is checked whether the one or more segregation effects are needed to be included in or excluded from the initial set of alloy compositions. Since the atomic radius of Ag is greater than that of other constituent elements, preferential elemental segregation at the surface is likely to occur in this alloy. This check of the inclusion or exclusion of segregation effects is carried out (or the analysis of the initial set of alloy compositions) by the system 100 and method of the present disclosure.
The inclusion of the one or more segregation effects in the initial set of alloy compositions comprises simulating the one or more surface atomic structures of the multi component alloy by using an interatomic potential based hybrid monte-carlo molecular dynamic technique, in an embodiment of the present disclosure. The inclusion of the one or more segregation effects in the initial set of alloy compositions is better understood by way of following description.

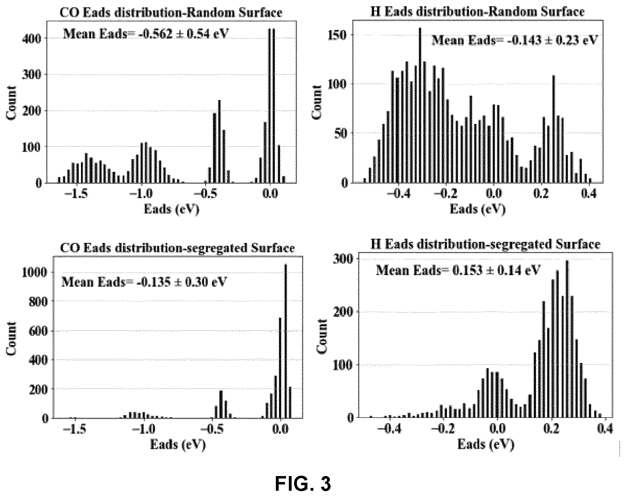

Multi component alloys are prone to elemental segregation at the surface atomic structures that significantly modifies both the chemical and structural diversity at the surface atomic structures. Since catalytic activity is intimately related to the surface microstructure, a knowledge of the atomic configuration at the surface is utmost essential for a correct evaluation of the alloy's efficiency as a catalyst. While a number of different interatomic potentials exit, the system 100 and method described herein chose the embedded atom potentials (EAM) to simulate surface segregation (or one or more surface atomic structures) in these alloys as EAM potentials are the most widely accepted choice for simulations of metals and alloys. It is to be understood by a person having ordinary skill in the art or person skilled in the art that such simulating techniques shall not be construed as limiting the scope of the present disclosure. Each simulation was carried out in an (x*y*z) supercell(s) size where values of x, y and z are 8, 8, and 6 respectively, wherein the supercell consisted of 384 atoms. An MC/MM protocol was used to 'evolve' the surfaces in these simulations wherein two different atoms in the system were swapped with a probability of $e^{\frac{-\Delta U}{k_B T}}$, where $\Delta U$ is the difference in the potential energies of the systems before and after the atom swap, $k_B$ is the Boltzmann constant and $T$ is the temperature. A total of 10 MC/MM swaps/atom was carried out for each system to identify thermodynamically most stable configurations of the system. In all cases, it was noticed that the surface was enriched in Ag and Au atoms. This contrasts with a purely random solid solution, wherein each element is equally likely to be present at the surface. The adsorption energy predictions using the trained ML model, shown in FIG. 3, clearly indicates the average adsorbate carbon monoxide (*CO) and Hydrogen (*H) Adsorption energies on both the surfaces is starkly different. Thus, inclusion of surface segregation as an inherent step in the screening Ag-Au-Cu-Pd-Pt containing catalysts is essential to identify realistic alloy compositions with potentially high activity and selectivity for CORR. More specifically, FIG. 3, with reference to FIGS. 1 through 2, depicts a carbon monoxide (CO) and Hydrogen (H) Adsorption energy distribution on the (111) random and segregated surface atomic structures of an equimolar AgAuCuPdPt alloy (also referred as random and segregated surface atomic structures of an equimolar AgAuCuPdPt alloy of orientation (111)), in accordance with an embodiment of the present disclosure.

The exclusion of one or more segregation effects in the initial set of alloy compositions comprises randomly arranging one or more atoms in the one or more surface atomic structures of the multi component alloy, in an embodiment of the present disclosure.

Having developed a ML model to predict the adsorbate adsorption energies and a protocol to obtain realistic surface microstructures with preferential element segregation, the next step is to optimize the composition of the alloy by including all these factors. Currently, the most active mono-metal catalyst for CORR is copper. However, it is plagued by poor product selectivity, one of the reasons for which is the occurrence of parasitic HER. Thus, the goal of the system 100 and method described herein is to search for an alloy that is at least as active as Cu for CORR but much worse than Cu in catalyzing the parasitic HER. Since CO and H adsorption energies are known to be excellent descriptors to qualify a catalyst for these two reactions, the quest for the system 100 and method described herein is to identify a catalyst with CO adsorption energy as close to Cu as possible (to maximize CORR activity), i.e., dE(CO) = abs($E_{ads}^{HEA}$(CO) - $E_{ads}^{Cu}$(CO)) ~= 0 and H adsorption energy that is much more unfavorable than Cu (to penalize HER activity), i.e., dE(H) = $E_{ads}^{HEA}$(H) - $E_{ads}^{Cu}$(H) » 0. In other words, the system and method have a multi-objective optimization problem with the two objective functions/optimization convergences, being *minimize dE(CO)* and *maximize dE(H).*

At step 208 of the method of the present disclosure, the one or more hardware processors 104 iteratively perform optimization of the composition of the multi component alloy. The step 208 pertaining to the optimization of the multi component alloy includes one or more steps. More specifically, at step 208a the one or more hardware processors 104 predict a set of relevant intermediate adsorption energies on the one or more surface atomic structures of the initial set of alloy compositions using one or more trained ML models based on the inclusion or the exclusion of one or more segregation effects in the initial set of alloy compositions. At step 208b the one or more hardware processors 104 identify one or more subsequent best candidate alloy compositions using the initial set of alloy compositions and the set of relevant predicted intermediate adsorption energies. These steps 208a and 208b are iteratively carried out until an optimization convergence is reached.

The step 208, including steps 208a and 208b, can be better understood by way of the following description. The one or more trained ML models are configured by the one or more hardware processors 104 to predict the average CO and H adsorption energies (averaged over 25 different atomic configurations for each alloy composition) on these segregated surface atomic structures. Thus, each point in the initial dataset (e.g., also referred as the initial set of alloy compositions and interchangeably used herein) was described using the 'm' dimension composition vector (e.g., 5 dimension composition vector) as features and the corresponding dE(CO) and dE(H) as the property values. The step 208b is better understood by way of following description. The catalyst composition optimization was carried out using a multi-objective Bayesian optimization (BO) technique using *BoTorch* package. Two different models were created using *SingleTaskGp* function in BoTorch, one each each for dE(CO) and dE(H) and these were combined using *ModelListGP* function. The loglikelihood was calculated using *SumMarginalLogLikelihood* while *qNoisyExpectedImprovement* was used as an acquisition function. Sampling of the composition space was carried out using *SobolQMCNormalSampler* with num_samples set at 10000 while the 'next best candidate' for successive iterations of BO was given by the *optimize_acqf_list* function. A gaussian process regression (GPR) model was used as the surrogate model in the BO procedure A surrogate Gaussian Process Regression (GPR) model was trained using the set of elements of step 202 and the trained GPR model was used to predict the property values on the 10,000 sampled drawn by *SobolQMCNormalSampler* (e.g., sampler as known in the art). Subsequently, the 'next best candidate' was identified based on the expected improvement scores.

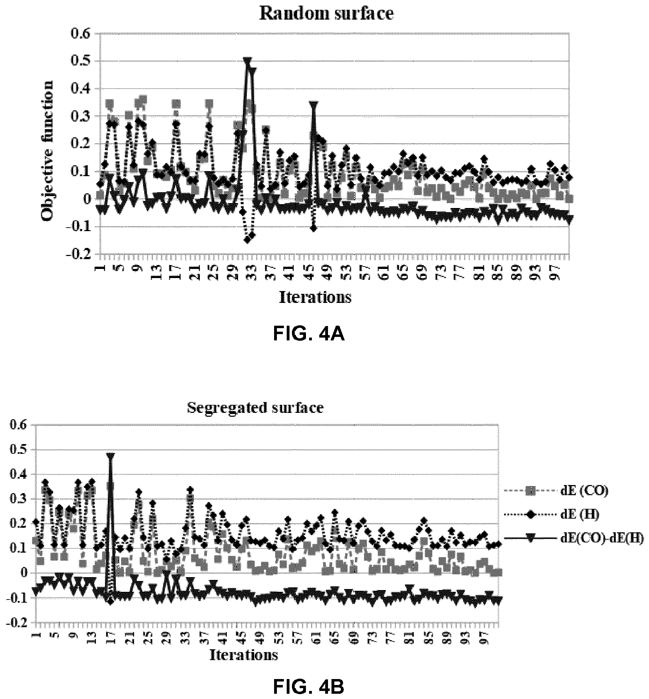

FIGS. 4A-4B, with reference to FIGS. 1 through 3, depict an evolution of one or more objective function values (also referred as optimization convergence) over the 100 Bayesian Optimization (BO) iterations for the random and segregated systems respectively, in accordance with an embodiment of the present disclosure. Once the iterations are completed, and the objective function is satisfied or the optimization convergence is reached, at step 210 of the method of the present disclosure, the one or more hardware processors 104 identify at least one best candidate alloy composition amongst the one or more subsequent best candidate alloy compositions as a multi component alloy catalyst that potentially maximizes activity and selectivity for the target chemical reaction. The step 210 is better understood by way of following description. Considering surface segregation, the multi component alloy that is likely to be the best candidate for CORR was found to have a composition of 22.3%Au-20.81%Cu-27.11%Pd-29.77%Pt. Excluding segregation effects, i.e., considering a random solid solution for this alloy (or random arrangement of the constituent atoms in the surface atomic structure), the best candidate had a composition of 37.74%Au - 35.15%Cu - 27.11%Pd. Interestingly, in both the cases (e.g., the inclusion and exclusion), the system 100 and method noted that the best alloy candidate was devoid of any Ag content. The above description may be better understood by way of following experimental results:

Experimental results:

**Case of CO reduction**

The system 100 and method identify new alloy compositions that are active and selective for CO reduction reaction (CORR). After following the steps that are provided in FIG. 2 the alloys that are having best activity and selectivity towards CORR over Hydrogen evolution reaction (HER) have been obtained. The best candidate alloy compositions for CORR in both random and segregated states are depicted in Table 3 below:

Table 3

| State | Alloy Composition |
|---|---|
| Random | 37.74%Au-35.15%Cu-27.11%Pd |
| Segregated | 22.3%Au-20.81%Cu-27.11%Pd-29.77%Pt |

Copper is the best mono-metal catalysts for CORR as outlined in the research. To achieve selective reduction of CO, the alloy must weakly bind to H and optimally binds CO. The alloys mentioned above optimally binds to CO (as good as copper) and weakly bind to H. The corresponding adsorption energy values for these alloys and pure copper are depicted in Table 4. More specifically, Table 4 illustrates comparison of CO and H adsorption energies of Cu and the best alloys found for CORR.

Table 4

| Metal/Alloy | $E^{CO}_{Ads}$ (eV) | $E^{H}_{Ads}$ (eV) |
|---|---|---|
| 100%Cu | -0.3735 | -0.0782 |
| 37.74%Au-35.15%Cu-27.11%Pd | -0.3737 | 0.0008 |
| 22.3%Au-20.81%Cu-27.11%Pd-29.77%Pt | -0.3726 | 0.0462 |

The difference in the CO adsorption energies of Cu and the best alloys is almost nil whereas the difference in the H binding energies is significantly wider. Hence, these alloys (or also referred as candidate alloy compositions) are likely to show better activity and selectivity towards CORR as compared to pure copper. Likewise, for any catalytic reaction the system and method of the present disclosure enable finding an alternative, more active and selective alloy (s) as a replacement of existing ones.

As mentioned above, conventional approaches fail to consider important effect of surface segregation on the catalytic activity. Preferential segregation of elements, which is a physical reality for many different alloys, significantly alters the surface microstructure. Catalytic activity is intimately related to the surface microstructure. Thus, optimization of such alloy compositions needs to include surface segregation as an integral part of the procedure. Embodiments of the present disclosure provide systems and methods that overcome this limitation by including surface segregation as an inherent part of catalyst composition optimization. At first, given the principal components of the alloy and a target chemical reaction, surface models of the alloy for diverse compositions and atomic configurations are created. Quantum chemical calculations (e.g., such as, but are not limited to, Density Function Theory (DFT), all electron calculations, and the like)) are then carried out on these surface atomic structures to compute the adsorption energy of the key reaction intermediates. Secondly, AI/ML models are trained to predict the adsorption energy of the key reaction intermediates. Later, instead of DFT calculations, the trained AI/ML models can be used to predict the adsorption energy of the intermediates on any new composition/configuration of the alloy. Interatomic potential based MC/MM simulations are carried out for observing the surface atomic structures of the multi component alloy and their evolvement to a thermodynamically stable state resulting from the preferential segregation of one or more elements to the surface of the alloy. At last, numerical optimization has been implemented by the system and method of the present disclosure to identify alloy compositions that maximize the activity and selectivity of the catalyst for the target chemical reaction. A mathematical function of the adsorption energies is used as the objective function (or optimization convergence) in numerical optimization. Since multicomponent alloys tend to undergo preferential elemental segregation, interatomic potential based monte-carlo/molecular mechanics (MC/MM) calculations/simulations have been carried out to identify such effects in alloy compositions identified during each iteration of the optimization procedure. The AI/MI, model is then used to predict the adsorption energies on the segregated surface atomic structures and the objective function/optimization convergence is evaluated using these values. The same procedure can also be followed without the inclusion of the MC/MM step, in which case, the system 100 identifies the best random solid solution composition formed from the multiple principal components. Thus, the catalyst screening as performed by the method of the present disclosure is physically more realistic compared to earlier reports of conventional approaches.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method, comprising:

   receiving, via one or more hardware processors, (i) a set of elements constituting a multi component alloy, and (ii) a target chemical reaction (202);
   generating, via the one or more hardware processors, an initial set of alloy compositions from the set of elements constituting the multi component alloy (204);
   analyzing, via the one or more hardware processors, the initial set of alloy compositions for the multi component alloy to determine an inclusion or an exclusion of one or more segregation effects in the initial set of alloy compositions (206);
   iteratively optimizing (208) the multi component alloy by:

   predicting, via the one or more hardware processors, a set of relevant intermediate adsorption energies on one or more surface atomic structures of the initial set of alloy compositions using one or more trained machine learning (ML) models based on the inclusion or the exclusion of the one or more segregation effects in the initial set of alloy compositions (208a); and
   identifying, via the one or more hardware processors, one or more subsequent best candidate alloy compositions using the initial set of alloy compositions and the set of relevant predicted intermediate adsorption energies (208b),

   until an optimization convergence is reached; and
   identifying, via the one or more hardware processors, at least one best candidate alloy composition amongst the one or more subsequent best candidate alloy compositions as a multi component alloy catalyst that potentially maximizes activity and selectivity for the target chemical reaction (210).

2. The processor implemented method as claimed in claim 1, wherein the one or more trained ML models are obtained by:

   identifying, via the one or more hardware processors, one or more relevant reaction intermediates for the target chemical reaction;
   computing, by using a quantum chemical technique via the one or more hardware processors, an adsorption energy of the one or more relevant reaction intermediates on one or more surface atomic structures of the multi component alloy; and
   training, via the one or more hardware processors, the one or more machine learning (ML) models using the adsorption energy of the one or more relevant reaction intermediates on the one or more surface atomic structures of the multi component alloy.

3. The processor implemented method as claimed in claim 1, wherein the inclusion of the one or more segregation effects in the initial set of alloy compositions comprises simulating the one or more surface atomic structures of the multi component alloy by using an interatomic potential based hybrid monte-carlo molecular dynamic technique.

**4.** The processor implemented method as claimed in claim 1, wherein the exclusion of one or more segregation effects in the initial set of alloy compositions comprises randomly arranging one or more atoms in the one or more surface atomic structures of the multi component alloy.

**5.** A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive (i) a set of elements constituting a multi component alloy, and (ii) a target chemical reaction;
generate an initial set of alloy compositions from the set of elements constituting the multi component alloy;
analyze the initial set of alloy compositions for the multi component alloy to determine an inclusion or an exclusion of one or more segregation effects in the initial set of alloy compositions;
iteratively optimize the multi component alloy by:

predicting a set of relevant intermediate adsorption energies on one or more surface atomic structures of the initial set of alloy compositions using the one or more trained machine learning (ML) models based on the inclusion or the exclusion of the one or more segregation effects in the initial set of alloy compositions; and
identifying one or more subsequent best candidate alloy compositions using the initial set of alloy compositions and the set of relevant predicted intermediate adsorption energies,

until an optimization convergence is reached; and
identify at least one best candidate alloy composition amongst the one or more subsequent best candidate alloy compositions as a multi component alloy catalyst that potentially maximizes activity and selectivity for the target chemical reaction.

**6.** The system as claimed in claim 5, wherein the one or more trained ML models are obtained by:

identifying, via the one or more hardware processors, one or more relevant reaction intermediates for the target chemical reaction;
computing, by using a quantum chemical technique via the one or more hardware processors, an adsorption energy of the one or more relevant reaction intermediates on one or more surface atomic structures of the multi component alloy; and
training, via the one or more hardware processors, one or more machine learning (ML) models using the adsorption energy of the one or more relevant reaction intermediates on the one or more surface atomic structures of the multi component alloy to obtain the one or more trained ML models.

**7.** The system as claimed in claim 5, wherein the inclusion of the one or more segregation effects in the initial set of alloy compositions comprises simulating the one or more surface atomic structures of the multi component alloy by using an interatomic potential based hybrid monte-carlo molecular dynamic technique.

**8.** The system as claimed in claim 5, wherein the exclusion of one or more segregation effects in the initial set of alloy compositions comprises randomly arranging one or more atoms in the one or more surface atomic structures of the multi component alloy.

**9.** One or more non-transitory machine-readable information storage mediums comprising one or more instructions which when executed by one or more hardware processors cause:

receiving (i) a set of elements constituting a multi component alloy, and (ii) a target chemical reaction;
generating an initial set of alloy compositions from the set of elements constituting the multi component alloy;
analyzing the initial set of alloy compositions for the multi component alloy to determine an inclusion or an exclusion of one or more segregation effects in the initial set of alloy compositions;
iteratively optimizing the multi component alloy by:

predicting a set of relevant intermediate adsorption energies on one or more surface atomic structures of the initial set of alloy compositions using one or more trained machine learning (ML) models based on the inclusion or the exclusion of the one or more segregation effects in the initial set of alloy compositions; and identifying one or more subsequent best candidate alloy compositions using the initial set of alloy compositions and the set of relevant predicted intermediate adsorption energies,

until an optimization convergence is reached; and
identifying at least one best candidate alloy composition amongst the one or more subsequent best candidate alloy compositions as a multi component alloy catalyst that potentially maximizes activity and selectivity for the target chemical reaction.

10. The one or more non-transitory machine-readable information storage mediums as claimed in claim 9, wherein the one or more trained ML models are obtained by:

identifying one or more relevant reaction intermediates for the target chemical reaction;
computing, by using a quantum chemical technique, an adsorption energy of the one or more relevant reaction intermediates on one or more surface atomic structures of the multi component alloy; and
training the one or more machine learning (ML) models using the adsorption energy of the one or more relevant reaction intermediates on the one or more surface atomic structures of the multi component alloy.

11. The one or more non-transitory machine-readable information storage mediums as claimed in claim 9, wherein the inclusion of the one or more segregation effects in the initial set of alloy compositions comprises simulating the one or more surface atomic structures of the multi component alloy by using an interatomic potential based hybrid monte-carlo molecular dynamic technique.

12. The one or more non-transitory machine-readable information storage mediums as claimed in claim 9, wherein the exclusion of one or more segregation effects in the initial set of alloy compositions comprises randomly arranging one or more atoms in the one or more surface atomic structures of the multi component alloy.

SYSTEM
100

MEMORY
102

DATABASE
108

HARDWARE
PROCESSOR(S)
104

INTERFACE(S)
106

FIG. 1

receiving (i) a set of elements constituting a multi component alloy and (ii) a target chemical reaction — 202

↓

generating an initial set of alloy compositions from the set of elements constituting the multi component alloy — 204

↓

analyzing the initial set of alloy compositions for the multi component alloy to determine an inclusion or an exclusion of segregation effect(s) in the initial set of alloy compositions — 206

↓

iteratively optimizing the multi component alloy

predicting a set of relevant intermediate adsorption energies on surface atomic structures of the initial set of alloy compositions using trained ML models based on the inclusion or the exclusion of the segregation effects in the initial set of alloy compositions — 208a

↓

identifying subsequent best candidate alloy composition(s) using the initial set of alloy compositions and the set of relevant predicted intermediate adsorption energies — 208b

until an optimization convergence is reached

— 208

↓

identifying at least one best candidate alloy composition amongst the subsequent best candidate alloy compositions as a multi component alloy catalyst that potentially maximizes activity and selectivity for the target chemical reaction — 210

**FIG. 2**

FIG. 3

EP 4 401 083 A2

**Random surface**

FIG. 4A

**Segregated surface**

FIG. 4B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321002360

**Non-patent literature cited in the description**

- **PEDERSEN et al.** *ACS Catal.,* 2020, vol. 10 (3), 2169-2176